# EUROPÄISCHE PATENTSCHRIFT

(11) **EP 2 036 491 B1**
(45) Veröffentlichungstag und Bekanntmachung des Hinweises auf die Patenterteilung: **28.03.2018**
(21) Anmeldenummer: 08400027.2
(22) Anmeldetag: 18.04.2008
(51) Int. Cl.: A61B 5/00, G06F 19/00

(54) **Beatmungsgerät und Verfahren zur Aktualisierung eines Beatmungsgerätes**
Lung ventilator and method for updating a lung ventilator
Respirateur artificiel et méthode pour actualiser un respirateur artificiel

(30) Priorität: 18.04.2007 DE 102007018587
(43) Veröffentlichungstag der Anmeldung: 18.03.2009
(60) Teilanmeldung: 11004763.6
(73) Patentinhaber: Weinmann Emergency Medical Technology GmbH + Co. KG, 22525 Hamburg (DE)
(72) Erfinder: Schöller, Bernd, 76135 Karlsruhe (DE); Marx, Thomas, 20144 Hamburg (DE); Elmar, Vitt, 27245 Rotenburg (DE)
(74) Vertreter: Klickow & Wetzel PartGmbB

(56) Entgegenhaltungen:
- EP-A- 1 702 649
- WO-A-01/54751
- WO-A-01/87149
- WO-A-2005/050525
- DE-A1-102005 049 643
- US-A1- 2002 183 642
- US-A1- 2005 137 487

## Beschreibung

Die Erfindung betrifft ein Verfahren zur Aktualisierung von einem Beatmungsgerät sowie ein Beatmungsgerät. Dabei wird ein im Gerät abgespeichertes Betriebsprogramm mindestens teilweise durch ein neues Betriebsprogramm ersetzt, sowie das Betriebsprogramm mindestens teilweise in einem veränderlichen Speicher des Gerätes gespeichert und bei der Durchführung der Aktualisierung mindestens teilweise durch das neue Betriebsprogramm ersetzt. Im Beatmungsgerät ist eine Schnittstelle (8) als USB-Schnittstelle realisiert, über die die Eingabe und/oder Ausgabe von Daten erfolgt und zudem sind im Beatmungsgerät Schnittstellen für adaptierbare Zusatzgeräte und Informations-Management-Systeme, beispielsweise zur Aufnahme von Speichermedien, oder zur Verbindung mit einem EKG, EEG, Drucker, Defibrillator, Pulsoximeter oder einem anderen medizintechnischen Gerät vorgesehen. Über ein Modem können aufgezeichnete Daten, wie Trends, außerordentliche Ereignisse, Warnmeldungen, dem Arzt; sowie Auffälligkeiten, Betriebsstunden oder andere zur Gewährleistung der einwandfreien Funktion, dem Nutzer oder dem Wartungs-/Kundendienst bei Bedarf übermittelt werden. Ein Pulsoximeter ist an eine Schnittstelle für die Ermittlung weiterer Körperparameter adaptierbar und die von dem adaptierten Pulsoximeter ermittelten Körperparameter können auf dem Display des Beatmungsgerätes angezeigt werden.

Die Erfindung betrifft darüber hinaus ein Beatmungsgerät mit einer Vorrichtung, bestehend aus einer internen oder externen Eingangsvorrichtung für Daten-Eingabe oder Daten-Empfang, aus einer hiermit mindestens mittelbar verbundenen Speichervorrichtung, mit in der Speichervorrichtung in einem Speicher abgelegten Daten oder Rechenalgorithmen, sowie mit Prüfmitteln, die eingegebene oder empfangene Daten mit in einem Speicher der Speichervorrichtung abgelegten Daten automatisch vergleichen, wobei Steuerungsmittel vorhanden sind, die aufgrund des durch die Prüfmittel ermittelten Vergleichsergebnisses nur bei bestimmten definierten Updates selbsttätig veranlassen, dass das Gerät dauerhaft oder zeitweise diese Daten, Programme auf Hardwarefunktionen anwendet, die diesen Updates zugeordnet sind, und dass die Ausführung dieser den Updates zugeordneten Hardwarefunktionen ohne das entsprechende Update durch diese Steuerungsmittel oder durch andere Mittel gesperrt ist, wobei das medizintechnische Gerät ein Beatmungsgerät ist und im Bereich eines Gerätegehäuses mit Bedienfeld sowie Anzeige (3) in einem Geräteinnenraum eine Atemgaspumpe angeordnet ist und über eine Kopplung ein Verbindungsschlauch angeschlossen ist, wobei das Gerätegehäuse zur Ermöglichung einer Datenübertragung eine Schnittstelle aufweist, sowie bei dem die Schnittstelle (8) als USB-Schnittstelle realisiert ist, über die die Eingabe und / oder Ausgabe von Daten erfolgt und zudem im Gerät Schnittstellen für adaptierbare Zusatzgeräte und Informations-Management-Systeme, beispielsweise zur Aufnahme von Speichermedien, oder zur Verbindung mit einem EKG, EEG, Drucker, Defibrillator, Pulsoximeter oder einem anderen medizintechnischen Gerät vorgesehen sind und über ein Modem können ebenfalls aufgezeichnete Daten, wie Trends, außerordentliche Ereignisse, Warnmeldungen dem Arzt; sowie Auffälligkeiten, Betriebsstunden oder andere zur Gewährleistung der einwandfreien Funktion dem Nutzer oder dem Wartungs-/Kundendienst bei Bedarf übermittelt werden und ein Pulsoximeter an eine Schnittstelle für die Ermittlung weiterer Körperparameter adaptierbar ist und die von dem adaptierten Pulsoximeter ermittelten Körperparameter auf der Anzeige des Beatmungsgerätes angezeigt werden.

Bei den meisten Medizintechnik-Geräten wird die Betriebssoftware in einem Permanentspeicher abgelegt und von einem *µ*Controller ausgeführt, der für diese Software ausgelegt ist. Folglich ist die Software in Form von nicht veränderbaren Programmanweisungen hinterlegt und der *µ*Controller ist nicht erweiterbar bzw. hat keine freien Kapazitäten. Im Falle eines Software-Updates müssen somit Speicherbauelemente ersetzt werden, was zur Folge hat, dass die Geräte von einem Fachmann demontiert werden müssen. Ein solches Software-Update ist aufwendig und teuer, da während der Dauer des Updateprozesses das betreffende Medizingerät nicht verwendet werden kann.

In anderen Fällen ist der Austausch von Hardware notwendig, um beispielsweise Sensoren mit erweiterter Funktionalität verwenden zu können.

Aufgrund der raschen Fortentwicklung der technischen Möglichkeiten sind Medizintechnik-Geräte häufig schon nach kurzer Nutzungsdauer nicht mehr auf dem Stand der Technik. Derzeit müssen Benutzer, die auf die neuesten oder weitere benötigte Funktionen angewiesen sind, daher regelmäßig ein neues Medizintechnik-Gerät erwerben.

In der DE 10 2005 049 643 A1 wird ein Beatmungsgerät mit einem Datenspeicher sowie einer zugeordneten Eingabemöglichkeit beschrieben. Das Beatmungsgerät wird von einer Betriebssoftware gesteuert. Ein Update kann durch Einstecken eines neuen Speichermoduls in vorgesehene freie Speicherplätze erfolgen. Über das Update ist es beispielsweise möglich, eine aktualisierte Betriebssoftware zu verwenden. Ein Update erfordert somit einen technischen Eingriff in das Gerät durch Ergänzung oder Austausch von Speicherbausteinen.

In der WO 05 050 525 A1 werden modular aufgebaute medizintechnische Geräte beschrieben. Die einzelnen Module werden jeweils von einer Betriebssoftware gesteuert. Eine Funktionsanpassung erfolgt durch Austausch oder Ergänzung kompletter Funktionsmodule.

Aus der WO 01 087 149 A1 ist ein weiteres modular aufgebautes medizintechnisches Gerät bekannt. Eine Mehrzahl von Systemmodulen sind über eine Datenfernübertragung mit einer zentralen Analyseeinheit koppelbar. Die Kopplung dient der Übertragung von Messinformationen.

In der US 02 0183 642 A1 wird ein unter Verwendung von Software gesteuertes Gerät zur Analyse von Lungengeräuschen eines Patienten beschrieben.

Aus der US 05 013 7487 A1 ist es bekannt, einen implantierten Sensor mit einem Auswertungssystem zur Messdatenübertragung zu koppeln.

Es wäre wünschenswert, für Medizintechnik-Geräte eine update Funktionalität vorzusehen, die es dem Benutzer ermöglicht, sein Medizintechnik-Gerät mit den jeweils neuesten oder zusätzlichen benötigten Funktionen aufzurüsten.

Ein Medizintechnik-Gerät ist im Sinne der Erfindung ein Beatmungsgerät. Andere Medizintechnik-Geräte, die nicht Teil der Erfindung sind, sind ein medizinisches, ärztliches oder der Gesundheit dienendes Gerät, insbesondere ein Meß-, Diagnose- oder Therapiegerät, wie beispielsweise ein Defibrillator, ein Sauerstoffkonzentrator, ein Pulsoximeter, ein Pulsspektrometer oder ein Diagnosegerät für Atmungsstörungen.

Aufgabe der vorliegenden Erfindung ist es, eine Vorrichtung der einleitend genannten Art derart zu konstruieren, dass die Vorrichtung für ein Medizintechnik-Gerät Hardware und/oder Softwareupdates durchführt, ohne dass dafür ein Hardwareaustausch erfolgen muss.

Diese Aufgabe wird erfindungsgemäß dadurch gelöst, dass im Bereich der Anzeige solche Bereiche vorgesehen sind, die erst dann aktiviert werden, wenn der betreffende Sensor adaptiert ist und in diesen Bereichen dann Messwerte des adaptierten Pulsoximeters angezeigt werden.

Insbesondere ist auch daran gedacht, dass die Vorrichtung für ein Medizintechnik-Gerät aus einer internen oder externen Eingangsvorrichtung für Dateneingabe und/oder Datenempfang besteht, sowie aus einer hiermit mindestens mittelbar verbundenen Speichervorrichtung, mit in der Speichervorrichtung in zumindest einem Speicher abgelegten Daten oder Rechen- bzw. Zählalgorithmen, sowie mit Prüfmitteln, die eingegebene oder empfangene Daten (hier: "Code") mit in einem oder mehreren Speichern der Speichervorrichtung abgelegten Daten automatisch vergleichen, insbesondere auch, nachdem oder während der Code und/oder die in einem oder mehreren Speichern abgelegten Daten verarbeitet wurden oder werden (insbesondere Rechen- bzw. Zählalgorithmen durchlaufen haben oder durchlaufen), wobei Steuerungsmittel vorhanden sind, die aufgrund des durch die Prüfmittel ermittelten Vergleichsergebnisses nur bei bestimmten definierten Code(s) selbsttätig veranlassen, dass das Gerät zumindest zeitweise bestimmte Funktionen ausführen kann, die diesen Codes zugeordnet sind, und dass die Ausführung dieser den Codes zugeordneten Funktionen ohne den entsprechenden Code durch diese Steuerungsmittel oder durch andere Mittel gesperrt ist.

Weitere Aufgabe der vorliegenden Erfindung ist es, ein Verfahren der einleitend genannten Art derart zu verbessern, dass eine einfache Aktualisierung des Gerätes unterstützt wird.

Diese Aufgabe wird erfindungsgemäß dadurch gelöst, dass im Bereich der Anzeige solche Bereiche vorgesehen sind, die erst dann aktiviert werden, wenn der betreffende Sensor adaptiert ist und in diesen Bereichen dann Messwerte des adaptierten Pulsoximeters angezeigt werden.

Gemäß der Erfindung besteht die Vorrichtung für ein Beatmungsgerät aus einer internen oder externen Eingangsvorrichtung für Daten- und/oder Rechenprogramm-Eingaben oder Daten- und/oder Rechenprogramm-Empfang, aus einer hiermit mindestens mittelbar verbundenen Speichervorrichtung, mit in der Speichervorrichtung in einem oder mehreren Speichern abgelegten Daten oder Rechen- bzw. Zählalgorithmen, sowie mit Prüfmitteln, die eingegebene oder empfangene Daten und/oder Rechenprogramme, hier als "Update" bezeichnet, mit in einem oder mehreren Speichern der Speichervorrichtung abgelegten Daten automatisch vergleichen, insbesondere auch, nachdem oder während das Update und/oder die in einem oder mehreren Speichern abgelegten Daten verarbeitet wurden oder werden und insbesondere Rechen- bzw. Zählalgorithmen durchlaufen haben oder durchlaufen, wobei Steuerungsmittel vorhanden sind, die aufgrund des durch die Prüfmittel ermittelten Vergleichsergebnisses nur bei bestimmten definierten Updates selbsttätig veranlassen, dass das Gerät dauerhaft oder zeitweise diese Daten, Programme oder hieraus berechnete Daten oder Programme auf Hardware-Weiter auf Seite 5 der Ursprungsunterlagen funktionen anwendet, die diesen Updates zugeordnet sind, und daß die Ausführung dieser den Updates zugeordneten Hardwarefunktionen ohne das entsprechende Update durch diese Steuerungsmittel oder durch andere Mittel gesperrt ist.

Betriebsprogramme sind beispielsweise die Betriebssoftware für die Gerätefunktion, Anwendungsprogramme, Auswertungsprogramme für Meßwerte oder ein Kommunikationsprogramm.

Gemäß der Erfindung besteht die Vorrichtung für ein Beatmungsgerät aus einer internen oder externen Eingangsvorrichtung für Dateneingaben oder Datenempfang, aus einer hiermit mindestens mittelbar verbundenen Speichervorrichtung, mit in der Speichervorrichtung in einem oder mehreren Speichern abgelegten Daten oder Rechen- bzw. Zählalgorithmen, sowie mit Prüfmitteln, die eingegebene oder empfangene Daten, hier: "Code", mit in einem oder mehreren Speichern der Speichervorrichtung abgelegten Daten automatisch vergleichen, insbesondere auch, nachdem oder während der Code und/oder die in einem oder mehreren Speichern abgelegten Daten verarbeitet wurden oder werden, wobei Steuerungsmittel vorhanden sind, die aufgrund des durch die Prüfmittel ermittelten Vergleichsergebnisses nur bei bestimmten definierten Code(s) selbsttätig veranlassen, daß das Gerät dauerhaft oder zeitweise mit einer oder mehreren bestimmten zusätzlichen internen oder externen Hardwarekomponenten zusammenarbeitet, die diesen Codes zugeordnet sind, und daß die Zusammenarbeit des Gerätes mit einer den Codes zugeordneten Hardwarekomponente ohne den entsprechenden Code durch diese Steuerungsmittel oder durch andere Mittel gesperrt ist.

Eine für den Benutzer optimierte Funktionalität wird dadurch erreicht, daß ein positives Vergleichsergebnis akustisch, optisch oder auf andere Weise dem Benutzer angezeigt oder gemeldet wird, z.B. als Erfolgsmeldung.

Eine für den Benutzer optimierte Funktionalität wird auch dadurch erreicht, daß ein negatives Vergleichsergebnis akustisch, optisch oder auf andere Weise dem Benutzer angezeigt oder gemeldet wird, z.B. als Fehlermeldung.

Eine erweiterte Funktionalität wird dadurch erreicht, daß in der Speichervorrichtung und/oder in dem Code Daten abgelegt sind, die, auch in verarbeiteter oder berechneter Form, die Ausführung der Funktionen und/oder deren Sperrung zeitlich in einer definierten Weise begrenzen.

Eine individuell anpassbare Verwendung wird dadurch erreicht, daß Hardwarekomponenten im Gerät enthalten sind, die ohne Code-Eingabe nicht in Funktion genommen werden können und insoweit für alle Funktionen gesperrt sind.

Eine konstruktiv einfache Realisierung wird dadurch erreicht, daß ohne die Code-Eingabe die Versorgung des Gerätes oder von Teilen hiervon mit elektrischer Energie (z.B. über Netzverbindung, Batterien oder Akkus) unterbunden, oder zeitmäßig oder leistungsmäßig in definierter Weise begrenzt wird.

Eine schnelle Realisierung der Freigabe wird dadurch erreicht, daß die Eingabe oder Übermittlung des Codes an die Eingangsvorrichtung mittels eines Datenträgers mit patienten- oder praxis- oder arztbezogenen Daten, wie über z.B. die Gesundheitskarte, oder über ein Lesegerät hierfür erfolgt.

Eine wirkungsvolle Sperrung wird dadurch erreicht, daß ohne die Code-Eingabe bestimmte Bauteile des Gerätes, insbesondere Sensoren bestimmter Kenndaten, zumindest zeitweise zur Nutzung für das Gerät gesperrt sind.

Eine erweiterte Funktionalität wird dadurch bereitgestellt, daß in der Speichervorrichtung und/oder in dem Code Daten abgelegt sind, die, auch in verarbeiteter oder berechneter Form, die Ausführung der den Updates zugeordneten Hardwarefunktionen und/oder deren Sperrung zeitlich in einer definierten Weise begrenzen.

Eine konstruktiv einfache Realisierung wird dadurch erreicht, daß Hardwarekomponenten im Gerät enthalten sind, die ohne das Update nicht in Funktion genommen werden können und insoweit für alle Funktionen gesperrt sind. In einer Ausführungsform sind ohne das Update bestimmte Bauteile des Gerätes, insbesondere Sensoren bestimmter Kenndaten, wie beispielsweise Wellenlängen, zumindest zeitweise zur Nutzung für das Gerät gesperrt. In einer Ausführungsform sind in der Speichervorrichtung und/oder in dem Code Daten abgelegt, die die Zusammenarbeit des Gerätes mit der zugeordneten zusätzlichen Hardwarekomponente und/oder deren Sperrung zeitlich in einer definierten Weise begrenzen. In einer Ausführungsform sind alternativ und / oder ergänzend bereits Hard- oder Softwarekomponenten im Gerät enthalten, die ohne die Zusammenarbeit des Gerätes mit der zugeordneten zusätzlichen Hardwarekomponente nicht in Funktion genommen werden können und insoweit für alle Funktionen gesperrt sind.

Eine gerätetechnische Ausführungsform der Erfindung ist dadurch realisiert, daß die ohne Code gesperrten, bereits vorhandenen Hardwarekomponenten und/oder die von zusätzlichen Hardwarekomponenten definierte Sensoren, insbesondere von bestimmter Wellenlänge sind.

Eine konstruktiv einfache Variante sieht vor, daß der Code in der zusätzlichen Hardwarekomponente und/oder in den Mitteln gespeichert ist, die die zusätzliche Hardwarekomponente mit dem Gerät verbinden und durch die Herstellung der Verbindung zwischen dieser Hardwarekomponente und dem Gerätes an die Eingangsvorrichtung gelangt.

Eine erhöhte Funktionalität wird dadurch bereitgestellt, daß eine optische oder akustische oder mechanische Anzeige/Signalausgabe vorhanden ist, die für die Freischaltung durch Code, das Upgrade oder die zusätzliche Hardwarekomponente Anzeige/Ausgabemöglichkeiten bereit hält, die ohne die Freischaltung durch Code, das Upgrade oder die zusätzliche Hardwarekomponente nicht genutzt werden können.

Ein Schutz vor unsachgemäßer Nutztung wird dadurch realisiert, daß eine definierte mehrmalige Eingabe eines oder mehrerer falscher Codes oder eines oder mehrerer nicht passender Updates zu einer zeitlich begrenzten oder unbegrenzten Sperre der Annahme jeder weiteren Eingabe für Codes oder Updates führt, welche ggf. nur mit einem weiteren Code entsperrt werden kann (der z. B. nur dem Hersteller bekannt ist). Ein Verfahren zur Aktualisierung von Software und/oder Firmware bei elektronischen Medizingeräten, wobei die Medizingeräte eine wiederbeschreibbare Speichereinheit aufweisen, zum Speichern einer aktualisierten und/oder einer vorhergehenden Version der Software und/oder Firmware, kann die folgenden Schritte enthalten:
(a) Bereitstellen der Software und/oder Firmware zur Aktualisierung mit Hilfe eines Aktualisierungsmittels;
(b) Kommunikation des Medizingerätes mit dem Aktualisierungsmittel;
(c) bei erfolgter Kommunikation: Übertragen der Aktualisierung vom Aktualisierungsmittel auf das Medizingerät;
(d) Schreiben der Aktualisierung in eine wiederbeschreibbare Speichereinheit;
(e) Verifizierung der Aktualisierung als gültige Version und Identifizierung der vorhergehenden Version als ungültig;
(f) Ausführen der aktualisierten Software und/oder Firmware.
Es ist daran gedacht, daß die vorhergehende Version mit der aktualisierten Software und/oder Firmware überschrieben oder in einen Restore-Speicher verschoben wird, insbesondere, wenn die aktualisierte Form nur für eine begrenzte Zeit freigeschaltet ist. Es ist auch daran gedacht, daß das Medizintechnikgerät zusätzlich einen Permanentspeicher einschließlich Anweisungen enthält, welche nach der Aktualisierung ausgeführt werden und woraufhin die wiederbeschreibbare Speichereinheit nach einer aktualisierten Software und/oder Firmware durchsucht wird und diese anschließend geladen und ausgeführt wird. Beschrieben wird auch ein Verfahren zur Aktualisierung von Software und/oder Firmware bei elektronischen Medizingeräten, wobei die Medizingeräte einen *µ*Controller mit einer Speichereinheit aufweisen und der *µ*Controller über eine Datenverbindung, beispielsweise über freie PINs, wie folgt angesteuert werden kann:
(a) Senden eines Signals an den *µ*Controller, um einen Reset des *µ*Controllers zu bewirken, beispielsweise über freie PINS;
(b) Bereitstellen eines Flash-Programms mit neuer Firmware zum Schreiben der neuen Firmware auf den *µ*Controller,
(c) wobei der Programmeintrittspunkt an eine definierte Stelle des *µ*Controllers geschrieben wird, welche nach Ende des Resets zuerst ausgelesen wird.

In der Zeichnung ist ein Beatmungsgerät schematisch dargestellt. Es zeigt:

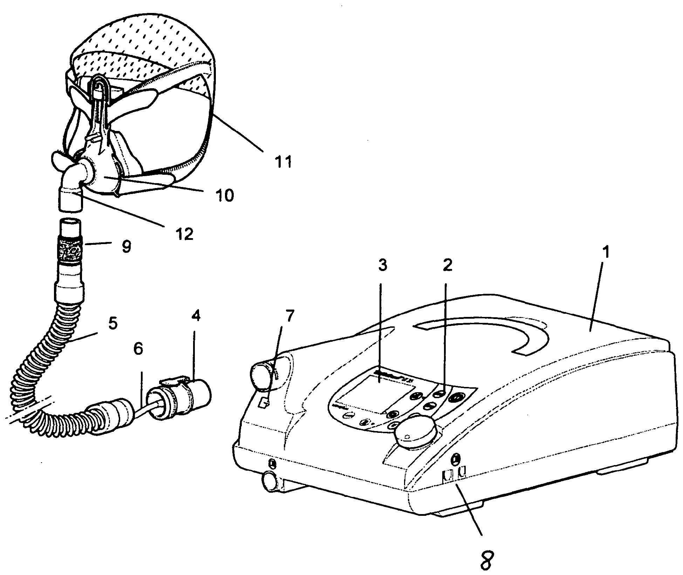

Fig. 1 eine perspektivische Darstellung eines Beatmungsgerätes mit Verbindungsschlauch zu einer Beatmungsmaske.

Fig. 1 zeigt den grundsätzlichen Aufbau eines erfindungsgemäßen Beatmungsgerätes. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf. Über die Schnittstelle können weitere Medizintechnische Geräte angeschlossen werden. Beispielsweise können Pulsoximeter oder Pulsspektrometer angeschlossen werden. Ein Anfeuchter kann ebenfalls adaptiert werden.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus ein als Beatmungsmaske (10) ausgebildetes Patienten-Interface, das als Nasalmaske realisiert ist. Eine Fixierung im Bereich des Kopfes eines Patienten kann über eine Kopfhaube (11) erfolgen. Im Bereich ihrer dem Verbindungsschlauch (5) zugewandten Ausdehnung weist das Patienten-Interface (10) ein Kupplungselement (12) auf.

Über die Schnittstelle (8) kann die Eingabe und / oder Ausgabe von Daten, wie beispielsweise eine Eingabe des Totraumvolumens oder ein Softwareupdate / Firmwareupdate, erfolgen. Die Schnittstellen können beispielsweise kabelgebunden, als Infrarot-Schnittstelle, als Bluetooth-Schnittstelle oder USB realisiert sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern.

Zudem können im Gerät Schnittstellen für adaptierbare Zusatzgeräte und Informations-Management-Systeme, beispielsweise zur Aufnahme von Speichermedien, oder zur Verbindung mit einem EKG, EEG, Drucker, Defibrillator, Pulsoximeter oder einem anderen medizintechnischen Gerät vorgesehen werden.

Über ein Modem oder eine andere Schnittstelle können ebenfalls aufgezeichnete Daten, wie Trends, außerordentliche Ereignisse, Warnmeldungen etc. dem Arzt, sowie Auffälligkeiten, Betriebsstunden oder andere zur Gewährleistung der einwandfreien Funktion dem Nutzer oder dem Wartungs-/Kundendienst bei Bedarf übermittelt werden.

Es ist ebenfalls möglich, über eine Schnittstelle Sensoren für die Ermittlung weiterer Körperparameter zu adaptieren. So können beispielsweise adaptierbare Zusatzgeräte wie EKG, EEG, EMG, EOG, Pulsoximeter, Pulsspektrometer ergänzt werden. Die von den adaptierten Sensoren ermittelten Körperparameter können auf dem Display des Beatmungsgerätes angezeigt werden. Dafür sind im Bereich des Displays solche Bereiche vorgesehen, die erst dann aktiviert werden, wenn der betreffende Sensor adaptiert ist. In diesen Bereichen werden dann Meßwerte der adaptierten Sensoren angezeigt. Ein solches Pulsoximeter ermittelt mittels zumindest zwei Wellenlängen die Sauerstoffsättigung SpO2 Zudem weist das Pulsoximeter eine weitere Wellenlänge auf, die der Ermittlung weiterer Parameter wie z.B. Methämoglobin oder Carboxyhämoglobin dienen kann, wobei diese Funktion in der aktuellen Konfiguration des Pulsoximeters nicht freigeschaltet ist und zur Freischaltung ein Code eingegeben werden muß und/oder ein erweiterter Rechen- bzw. Zählalgorithmus aufgespielt werden muß. Die Vorrichtung besteht aus einer Eingangsvorrichtung für Dateneingabe / Datenempfang, insbesondere zum Empfang von Updates oder Aktualisierungen der Rechen- bzw. Zählalgorithmen des Pulsoximeters, und aus einer hiermit verbundenen Speichervorrichtung mit in wenigstens einem Speicher abgelegten, Rechen- bzw. Zählalgorithmen, die z.B. der Ermittlung zumindest folgender Meßparameter dienen: SpO2 und Pulsfrequenz.

Über die Eingangsvorrichtung für Dateneingabe / Datenempfang, beispielsweise als Tastenfeld ausgeführt, kann ein Code eingegeben werden, der der Freigabe von Gerätefunktionen dient.

Prüfmittel vergleichen den eingegebenen Code mit einem in einem Speicher der Speichervorrichtung abgelegten Vergleichscode zur Verifikation des Codes, wobei Steuerungsmittel aufgrund des durch die Prüfmittel ermittelten Vergleichsergebnisses nur bei bestimmten definierten Code(s) selbsttätig veranlassen, daß das Pulsoximeter zumindest zeitweise bestimmte Funktionen ausführen kann, nämlich die Ermittlung der erweiterten Meßgrößen (z.B. Methämoglobin oder Carboxyhämoglobin) unter Verwendung von zumindest drei Wellenlängen, die diesen Codes zugeordnet sind. Die Ausführung dieser den Codes zugeordneten Funktionen ist ohne den entsprechenden Code durch die Steuerungsmittel oder durch andere Mittel gesperrt.

Nach der Codeingabe wird ein positives Vergleichsergebnis akustisch oder optisch, beispielsweise durch eine grün leuchtende LED, dem Benutzer als Erfolgsmeldung gemeldet.

Ein negatives Vergleichsergebnis wird akustisch oder optisch, beispielsweise durch eine rot leuchtende LED, dem Benutzer als Fehlermeldung gemeldet. Es ist vorgesehen, daß der Sensor des Pulsoximeters bereits für die Ermittlung zusätzlicher Meßparameter wie Methämoglobin und/oder Carboxyhämoglobin und/oder Hämoglobinkonzentration ausgerüstet ist, beipsielsweise dadurch, dass zumindest drei verschieden Wellenlängen emittiert werden können. Für die Ermittlung von SpO2 werden davon zwei Wellenlängen verwendet. Durch eine Code-Eingabe wird zumindest eine weitere Wellenlänge aktiviert, beispielsweise dadurch, dass die betreffende LED etwa der Energieversorgung in Funktion genommen wird und entsprechend einer Rechen- bzw. Zählalgorithmus die Daten von zumindest drei verschiednen Wellenlängen auswertet zur Ermittlung von SpO2 sowie der weiteren Meßparameter, wie z.B. Methämoglobin und/oder Carboxyhämoglobin und/oder Hämoglobinkonzentration. Es wird ohne die Code-Eingabe die Versorgung von zusätzlichen LED's mit elektrischer Energie unterbunden. Dieses oder ein anders Pulsoximeter weist zumindest drei Wellenlängen im Bereich 500 nm bis 950 nm auf, die der Ermittlung der Parameter Methämoglobin oder Carboxyhämoglobin und SpO2 und Pulsfrequenz dienen. Zudem weist der Sensor des Pulsoximeters zumindest eine weitere Wellenlänge im Bereich 950 bis 2500 nm auf, die der Ermittlung der Hämoglobinkonzentration dienen kann, wobei diese Funktion in der aktuellen Konfiguration des Pulsoximeters nicht freigeschaltet ist.

Zur Freischaltung der weiteren Wellenlänge im Bereich 950 bis 2500 nm, die der Ermittlung der Hämoglobinkonzentration dient, muß ein Code eingegeben werden. Durch den Code wird die entsprechende LED und/oder ein erweiterter Rechen- bzw. Zählalgorithmus freigeschaltet, und zwar zur Ermittlung der cHb aus zumindest vier Wellenlängen im Bereich 500 bis 2500 nm. Dieses oder ein anders Pulsoximeter weist zumindest drei Wellenlängen im Bereich 500 nm bis 950 nm auf, die der Ermittlung der Parameter Methämoglobin oder Carboxyhämoglobin und SpO2 und Pulsfrequenz dienen. Zudem weist der Sensor des Pulsoximeters zumindest eine weitere Wellenlänge im Bereich 950 bis 2500 nm auf, die der Ermittlung der Hämoglobinkonzentration cHb dient. Zur Ermittlung, beispielsweise der Hämoglobinkonzentration, wird ein erweiterter Rechen- bzw. Zählalgorithmus über die Eingangsvorrichtung für Dateneingabe / Datenempfang aufgespielt und in der hiermit verbundenen Speichervorrichtung in einem Speicher abgelegt, wobei der Programmeintrittspunkt des erweiterten Rechen- bzw. Zählalgorithmus an eine definierte Stelle des Speichers geschrieben wird, welche zuerst ausgelesen wird.

Bevorzugt wird der bisher vorhandene Rechen- bzw. Zählalgorithmus, der der Ermittlung der Parameter Methämoglobin oder Carboxyhämoglobin und SpO2 und Pulsfrequenz diente, überschrieben oder deaktiviert. Eine Vorrichtung für ein Pulsoximeter besteht aus einer Eingangsvorrichtung für ein Update und aus einer hiermit verbundenen Speichervorrichtung, mit einem in der Speichervorrichtung in einem Speicher abgelegten Rechen- bzw. Zählalgorithmus, der der Ermittlung der Parameter SpO2 und Methämoglobin und/oder Carboxyhömoglobin unter Auswertung der Daten von zumindest drei Wellenlängen aus dem Bereich 500 bis 950 nm, dient, wobei ein Prüfmittel das Update mit in den Speichern der Speichervorrichtung abgelegten Daten automatisch vergleicht, und ein Steuerungsmittel aufgrund des durch die Prüfmittel ermittelten Vergleichsergebnisses nur bei Verifizierung des Updates veranlasst, daß das Pulsoximeter das Update ausführt, wobei das Update der Ermittlung des Parameters Hämoglobinkonzentration unter Auswertung der Daten von zumindest vier Wellenlängen aus dem Bereich 500 bis 2500 nm dient.

Die Vorrichtung kann alternativ für ein Pulsoximeter realisiert sein, welches der Ermittlung der Parameter SpO2 und Pulsfrequenz dient. Dazu verwendet das Pulsoximeter einen Sensor mit zwei aktiven LEDs, die der Bestimmung von SpO2 dienen. Zusätzlich weist der Sensor zumindest eine nicht aktive, aber aktivierbare LED auf, die der Bestimmung zumindest eines weiteren Parameters, (ausgewählt aus der Gruppe: Methämoglobin, Carboxyhämoglobin, Hämoglobinkonzentration, Bilirubin, Glucose) dient.

Das Pulsoximeter weist einen Rechen- bzw. Zählalgorithmus auf, der der Bestimmung von SpO2 und Pulsfrequenz dient und zusätzlich zumindest einen weiteren Parameter, (ausgewählt aus der Gruppe: Methämoglobin, Carboxyhämoglobin, Hämoglobinkonzentration, Bilirubin, Glucose) ermitteln kann. Das Pulsoximeter weist weiterhin eine Eingangsvorrichtung für Dateneingaben auf, mit einem hiermit verbundenen Prüfmittel.

Zur Aktivierung der nicht genutzten LED und/oder des Rechen- bzw. Zählalgorithmus zur Bestimmung von zusätzlich zumindest einem weiteren Parameter (ausgewählt aus der Gruppe: Methämoglobin, Carboxyhämoglobin, Hämoglobinkonzentration, Bilirubin, Glucose) wird ein Code über die Eingangsvorrichtung für Dateneingabe eingegeben und durch das Prüfmittel mit den in einem Speicher der Speichervorrichtung abgelegten Daten verglichen. Ein Steuerungsmittel veranlasst, aufgrund des durch die Prüfmittel ermittelten Vergleichsergebnisses, die Aktivierung der nicht genutzten LED und/oder des Rechen- bzw. Zählalgorithmus zur Bestimmung von zusätzlich zumindest einem weiteren Parameter (ausgewählt aus der Gruppe: Methämoglobin, Carboxyhämoglobin, Hämoglobinkonzentration, Bilirubin, Glucose), wenn der eingegebene Code durch das Prüfmittel verifiziert wurde. Das Pulsoximeter weist ein Display auf, welches Anzeige-/Ausgabemöglichkeiten bereit hält, die erst nach Freischaltung durch einen Code oder nach der Durchführung des Upgrades freigegeben werden. So werden im Betrieb vor Durchführung des Updates beispielsweise die Parameter SpO2 und Pulsfrequenz angezeigt. Nach der Durchführung des Updates sind im Bereich des Display Anzeige/Ausgabe aktiviert zur Anzeige der Parameter Methämoglobin und/oder Carboxyhämoglobin und/oder Hämoglobinkonzentration, und/oder Bilirubin und/oder Glucose, beispielsweise in der Form: SaMet, SaCO, SaO2, cHb, wobei die cHb und die Glucose in g/dl angezeigt wird und beispielsweise die SaMet, SaCO in % dargestellt werden. Hierbei handelt es sich nur um einige der denkbaren Anwendungsmöglichkeiten, die sich ebenso auf andere Parameter beziehen können.

Zum Beispiel liegt der Anzeigebereich für die Konzentration des Hämoglobins cHb im Bereich 5 bis 25 g/dl. Alternativ oder ergänzend ist eine Anzeige in mmol/l vorgesehen. Der Anzeigebereich für einen Kohlenmonoxidanteil SaCO im Blut liegt bei 0 % bis 60 % Sättigung. Es ist es auch möglich, ein Update für die Ermittlung des Parametres Bilirubin durchzuführen. Das Update erfolgt ausgehend von zumindest zwei verschiedenen Wellenlängen, ausgewählt aus der Gruppe 300nm ± 15%, 400nm ± 15%, 460 nm ± 15%, 480 nm ± 15%, 520 nm ± 15%, 550 nm ± 15%, 560 nm ± 15%, 606 nm ± 15%, 617 nm ± 15%, 620 nm ± 15%, 630 nm ± 15%, 650 nm ± 15%, 660 nm ±, 705 nm ± 15%, 710 nm ± 15%, 720 nm ±1 0%, 805 nm ± 15%, 810 nm ± 15%, 880 nm ± 15%, 905 nm ± 15%, 910 nm ± 15%, 950 nm ± 15%, 980 nm ± 15%, 980 nm ± 15%, 1050 nm ± 15%, 1200 nm ± 15%, 1310 nm ± 15%, 1380 nm ± 15%, 1450 nm ± 15%, 1600 nm ± 15%, 1800 nm ± 15%, 2000nm ± 15%, 2500nm ± 15% auf zumindest drei verschiedenen Wellenlängen, ausgewählt aus der Gruppe 300nm ± 15%, 400nm ± 15%, 460 nm ± 15%, 480 nm ± 15%, 520 nm ± 15%, 550 nm ± 15%, 560 nm ± 15%, 606 nm ± 15%, 617 nm ± 15%, 620 nm ± 15%, 630 nm ± 15%, 650 nm ± 15%, 660 nm ±, 705 nm ± 15%, 710 nm ± 15%, 720 nm ±1 0%, 805 nm ± 15%, 810 nm ± 15%, 880 nm ± 15%, 905 nm ± 15%, 910 nm ± 15%, 950 nm ± 15%, 980 nm ± 15%, 980 nm ± 15%, 1050 nm ± 15%, 1200 nm ± 15%, 1310 nm ± 15%, 1380 nm ± 15%, 1450 nm ± 15%, 1600 nm ± 15%, 1800 nm ± 15%, 2000nm ± 15%, 2500nm ± 15%. Es ist ebenfalls daran gedacht, ausgehend von zumindest zwei Wellenlängen, über ein update auf zumindest vier verschiedene Wellenlängen zu erweitern, ausgewählt aus der Gruppe 300nm ± 15%, 400nm ± 15%, 460 nm ± 15%, 480 nm ± 15%, 520 nm ± 15%, 550 nm ± 15%, 560 nm ± 15%, 606 nm ± 15%, 617 nm ± 15%, 620 nm ± 15%, 630 nm ± 15%, 650 nm ± 15%, 660 nm ±, 705 nm ± 15%, 710 nm ± 15%, 720 nm ±1 0%, 805 nm ± 15%, 810 nm ± 15%, 880 nm ± 15%, 905 nm ± 15%, 910 nm ± 15%, 950 nm ± 15%, 980 nm ± 15%, 980 nm ± 15%, 1050 nm ± 15%, 1200 nm ± 15%, 1310 nm ± 15%, 1380 nm ± 15%, 1450 nm ± 15%, 1600 nm ± 15%, 1800 nm ± 15%, 2000nm ± 15%, 2500nm ± 15%.

Zur Bestimmung der Hämoglobinkonzentration cHb ist beispielsweise vorgesehen, ausgehend von zumindest zwei Wellenlängen, über ein update auf zumindest drei Wellenlängen zu erweitern, wobei zumindest bei einer Wellenlänge eine hohe Absorption für Wasser vorliegt ausgewählt aus der Gruppe 950 nm ± 15%, 980 nm ± 15%, 980 nm ± 15%, 1050 nm ± 15%, 1200 nm ± 15%, 1310 nm ± 15%, 1380 nm ± 15%, 1400 nm ± 15%, 1450 nm ± 15%, 1500 nm ± 15%, 1550 nm ± 15%, 1600 nm ± 15%, 1700 nm ± 15%, 1800 nm ± 15%, 1900 nm ± 15%, 2000nm ± 15%, 2500nm ± 15%. Es ist auch daran gedacht, ein medizinisches, ärztliches oder der Gesundheit dienendes Gerät, insbesondere ein Meß-, Diagnose- oder Therapiegerät, über eine Schnittstelle zumindest zeitweise mit einem adaptierbaren Zusatzgerät zu verbinden, beispielsweise mit einem EKG, EEG, EOG, EMG, Drucker, Monitor, Defibrillator, Pulsoximeter oder einem anderen medizintechnischen Gerät. Hierbei ist ebenfalls eine Freigabe des adaptierbaren Zusatzgerätes durch eine Code-Eingabe vorgesehen.

## Patentansprüche

1. Verfahren zur Aktualisierung eines Beatmungsgerätes,
bei dem ein im Gerät abgespeichertes Betriebsprogramm mindestens teilweise durch ein neues Betriebsprogramm ersetzt wird und das Betriebsprogramm mindestens teilweise in einem veränderlichen Speicher des Gerätes gespeichert wird und bei der Durchführung der Aktualisierung mindestens teilweise durch das neue Betriebsprogramm ersetzt wird, wobei
im Beatmungsgerät im Bereich eines Gerätegehäuses (1) mit Bedienfeld (2) sowie einem Display als Anzeige (3) in einem Geräteinnenraum eine Atemgaspumpe angeordnet ist und über eine Kopplung (4) ein Verbindungsschlauch (5) angeschlossen ist, wobei
das Gerätegehäuse (1) zur Ermöglichung einer Datenübertragung eine Schnittstelle (8) aufweist und wobei die Schnittstelle (8) als USB-Schnittstelle realisiert ist, über die die Eingabe und/oder Ausgabe von Daten erfolgt und zudem im Gerät Schnittstellen für adaptierbare Zusatzgeräte und Informations-Management-Systeme, beispielsweise zur Aufnahme von Speichermedien, oder zur Verbindung mit einem EKG, EEG, Drucker, Defibrillator, Pulsoximeter oder einem anderen medizintechnischen Gerät vorgesehen sind und wobei
über ein Modem aufgezeichnete Daten, wie Trends, außerordentliche Ereignisse, Warnmeldungen, dem Arzt; sowie Auffälligkeiten, Betriebsstunden oder andere zur Gewährleistung der einwandfreien Funktion, dem Nutzer oder dem Wartungs-/Kundendienst bei Bedarf übermittelt werden, und wobei
ein Pulsoximeter an eine Schnittstelle für die Ermittlung weiterer Körperparameter adaptierbar ist und die von dem adaptierten Pulsoximeter ermittelten Körperparameter auf dem Display des Beatmungsgerätes angezeigt werden,
**dadurch gekennzeichnet, dass**
im Bereich des Displays solche Bereiche vorgesehen sind, die erst dann aktiviert werden, wenn der betreffende Sensor adaptiert ist und in diesen Bereichen dann Messwerte des adaptierten Pulsoximeters angezeigt werden.

2. Verfahren nach Anspruch 1, **dadurch gekennzeichnet, dass** das neue Betriebsprogramm auf einem Datenträger gespeichert und von einem Lesegerät zum Speicher übertragen wird.

3. Verfahren nach Anspruch 1, **dadurch gekennzeichnet, dass** das neue Betriebsprogramm online zum Gerät übertragen wird.

4. Verfahren nach einem der Ansprüche 1 bis 3, **dadurch gekennzeichnet, dass** im Bereich des Gerätes ein nicht bei der Aktualisierung veränderbares Basisbetriebsprogramm gespeichert wird.

5. Verfahren nach einem der Ansprüche 1 bis 4, **dadurch gekennzeichnet, dass** von einem Prüfmittel des Gerätes ein eingegebener Code mit einem Referenzwert verglichen und mindestens eine Funktion des Gerätes nur bei einer Übereinstimmung des Codes mit dem Referenzwert freigeschaltet wird.

6. Verfahren nach einem der Ansprüche 1 bis 5, **dadurch gekennzeichnet, dass** durch das neue Betriebsprogramm bereits im Gerät vorhandene Hardware-Komponenten aktiviert werden.

7. Verfahren nach einem der Ansprüche 1 bis 6, **dadurch gekennzeichnet, dass** durch das neue Betriebsprogramm ein Funktionalität von bereits vorhandenen Hardware-Komponenten des Gerätes verändert wird.

8. Verfahren nach einem der Ansprüche 1 bis 7, **dadurch gekennzeichnet, dass** ein positives Vergleichsergebnis akustisch, optisch oder auf andere Weise dem Benutzer angezeigt oder gemeldet wird.

9. Verfahren nach einem der vorhergehenden Ansprüche, **dadurch gekennzeichnet, dass** ein negatives Vergleichsergebnis akustisch, optisch oder auf andere Weise dem Benutzer angezeigt oder gemeldet wird.

10. Verfahren nach einem der vorhergehenden Ansprüche, **dadurch gekennzeichnet, dass** in der Speichervorrichtung und/oder in dem Code Daten abgelegt sind, die (auch in verarbeiteter oder berechneter Form) die Ausführung der Funktionen und/oder deren Sperrung zeitlich in einer definierten Weise begrenzen.

11. Verfahren nach einem der vorhergehenden Ansprüche, **dadurch gekennzeichnet, dass** Hardwarekomponenten im Gerät enthalten sind, die ohne Code-Eingabe nicht in Funktion genommen werden können und insoweit für alle Funktionen gesperrt sind.

12. Verfahren nach einem der vorhergehenden Ansprüche, **dadurch gekennzeichnet, dass** ohne die Code-Eingabe die Versorgung des Gerätes oder von Teilen hiervon mit elektrischer Energie (z.B. über Netzverbindung, Batterien oder Akkus) unterbunden, oder zeitmäßig oder leistungsmäßig in definierter Weise begrenzt wird.

13. Verfahren nach einem der vorhergehenden Ansprüche, **dadurch gekennzeichnet, dass** die Eingabe oder Übermittlung des Codes an die Eingangsvorrichtung mittels eines Datenträgers mit patienten- oder praxis- oder arztbezogenen Daten (z.B. Gesundheitskarte) oder über ein Lesegerät hierfür (auch drahtlos) erfolgt.

14. Verfahren nach einem der vorhergehenden Ansprüche, **dadurch gekennzeichnet, dass** die Eingabe oder Übermittlung des Codes an die Eingangsvorrichtung durch eine (auch drahtlose) Vorrichtung erfolgt, die einem anderen medizinischen, ärztlichen oder gesundheitsbezogenen Geräte zumindest zeitweise intern oder extern zugeordnet ist oder die als Schnittstelle mehrere medizinische, ärztliche oder gesundheitsbezogene Geräte über zumindest zeitweisen Daten- oder Programmaustausch verbindet.

15. Verfahren nach einem der vorhergehenden Ansprüche, **dadurch gekennzeichnet, dass** ohne die Code-Eingabe bestimmte Bauteile des Gerätes, insbesondere Sensoren bestimmter Kenndaten zumindest zeitweise zur Nutzung für das Gerät gesperrt sind.

16. Beatmungsgerät mit einer Vorrichtung,
wobei die Vorrichtung besteht aus einer internen oder externen Eingangsvorrichtung für Daten-Eingabe oder Daten-Empfang, aus einer hiermit mindestens mittelbar verbundenen Speichervorrichtung, mit in der Speichervorrichtung in einem Speicher abgelegten Daten oder Rechenalgorithmen, sowie mit Prüfmitteln, die eingerichtet sind, um eingegebene oder empfangene Daten mit in einem Speicher der Speichervorrichtung abgelegten Daten automatisch zu vergleichen, wobei Steuerungsmittel vorhanden sind, die aufgrund des durch die Prüfmittel ermittelten Vergleichsergebnisses nur bei bestimmten definierten Updates selbsttätig veranlassen, dass das Beatmungsgerät dauerhaft oder zeitweise diese Daten auf Hardwarefunktionen anwendet, die diesen Updates zugeordnet sind, und dass die Ausführung dieser den Updates zugeordneten Hardwarefunktionen ohne das entsprechende Update durch diese Steuerungsmittel oder durch andere Mittel gesperrt ist, wobei
in dem Beatmungsgerät im Bereich eines Gerätegehäuses (1) mit Bedienfeld (2) sowie einem Display als Anzeige (3) in einem Geräteinnenraum eine Atemgaspumpe angeordnet ist und über eine Kopplung (4) ein Verbindungsschlauch (5) angeschlossen ist, wobei
das Gerätegehäuse (1) zur Ermöglichung einer Datenübertragung eine Schnittstelle (8) aufweist und wobei die Schnittstelle (8) als USB-Schnittstelle realisiert ist, die eingerichtet ist, die Eingabe und / oder Ausgabe von Daten erfolgen zu lassen und wobei zudem im Beatmungsgerät Schnittstellen für adaptierbare Zusatzgeräte und Informations-Management-Systeme, beispielsweise zur Aufnahme von Speichermedien, oder zur Verbindung mit einem EKG, EEG, Drucker, Defibrillator, Pulsoximeter oder einem anderen medizintechnischen Gerät vorgesehen sind und
mit einem Modem, das eingerichtet ist, um aufgezeichnete Daten, wie Trends, außerordentliche Ereignisse, Warnmeldungen dem Arzt; sowie Auffälligkeiten, Betriebsstunden oder andere zur Gewährleistung der einwandfreien Funktion dem Nutzer oder dem Wartungs-/Kundendienst bei Bedarf übermitteln zu können, und wobei
ein Pulsoximeter an eine Schnittstelle für die Ermittlung weiterer Körperparameter adaptierbar ist und wobei
das Display eingerichtet ist, um die von dem adaptierten Pulsoximeter ermittelten Körperparameter anzuzeigen,
**dadurch gekennzeichnet, dass**
im Bereich des Displays solche Bereiche vorgesehen sind, die erst dann aktiviert werden, wenn der betreffende Sensor adaptiert ist und in diesen Bereichen dann Messwerte des adaptierten Pulsoximeters angezeigt werden.

17. Beatmungsgerät nach einem der vorhergehenden Ansprüche, **dadurch gekennzeichnet, dass** Hardwarekomponenten im Beatmungsgerät enthalten sind, die ohne das Update nicht in Funktion genommen werden können und insoweit für alle Funktionen gesperrt sind.

18. Beatmungsgerät nach einem der vorhergehenden Ansprüche, **dadurch gekennzeichnet, dass** ohne das Update die Versorgung des Beatmungsgerätes oder von Teilen hiervon mit elektrischer Energie (z.B. über Netzverbindung, Batterien oder Akkus) unterbunden, oder zeitmäßig oder leistungsmäßig in definierter Weise begrenzt wird.

19. Beatmungsgerät nach einem der vorhergehenden Ansprüche, **dadurch gekennzeichnet, dass** die Eingabe oder Übermittlung des Updates an die Eingangsvorrichtung mittels eines Datenträgers mit patienten- oder praxis- oder arztbezogenen Daten (z.B. Gesundheitskarte) oder über ein Lesegerät hierfür (auch drahtlos) erfolgt.

20. Beatmungsgerät nach einem der vorhergehenden Ansprüche, **dadurch gekennzeichnet, dass** die Eingabe oder Übermittlung des Updates an die Eingangsvorrichtung durch eine beispielsweise drahtlose Vorrichtung erfolgt, die einem anderen medizinischen, ärztlichen oder gesundheitsbezogenen Geräte zumindest zeitweise intern oder extern zugeordnet ist oder die als Schnittstelle mehrere medizinische, ärztliche oder gesundheitsbezogene Geräte über zumindest zeitweisen Daten- oder Programmaustausch verbindet.

21. Beatmungsgerät nach einem der vorhergehenden Ansprüche, **dadurch gekennzeichnet, dass** ohne das Update bestimmte Bauteile des Gerätes, insbesondere Sensoren bestimmter Kenndaten zumindest zeitweise zur Nutzung für das Gerät gesperrt sind.

22. Beatmungsgerät nach Anspruch 21, **dadurch gekennzeichnet, dass** die Kenndaten die Wellenlängen der Sensoren sind.

23. Beatmungsgerät nach einem der vorhergehenden Ansprüche, **dadurch gekennzeichnet, dass** bereits Hard- oder Softwarekomponenten im Beatmungsgerät enthalten sind, die ohne die Zusammenarbeit des Gerätes mit der zugeordneten zusätzlichen Hardwarekomponente nicht in Funktion genommen werden können und insoweit für alle Funktionen gesperrt sind.

24. Beatmungsgerät nach einem der vorhergehenden Ansprüche, **dadurch gekennzeichnet, dass** ohne die Zusammenarbeit des Gerätes mit der zugeordneten zusätzlichen Hardwarekomponente die Versorgung des Gerätes oder von Teilen hiervon mit elektrischer Energie (z.B. über Netzverbindung, Batterien oder Akkus) unterbunden, oder zeitmäßig oder leistungsmäßig in definierter Weise begrenzt wird.

25. Beatmungsgerät nach Anspruch 24, **dadurch gekennzeichnet, dass** die ohne Code gesperrten, bereits vorhandenen Hardwarekomponenten definierte Sensoren, insbesondere von bestimmter Wellenlänge, sind.

26. Beatmungsgerät nach einem der vorhergehenden Ansprüche, **dadurch gekennzeichnet, dass** eine optische Anzeige vorhanden ist, die für die Freischaltung durch Code, das Update oder die zusätzliche Hardwarekomponente Anzeigemöglichkeiten bereit hält, die ohne die Freischaltung durch Code, das Update oder die zusätzliche Hardwarekomponente nicht genutzt werden.

27. Beatmungsgerät nach einem der vorhergehenden Ansprüche, **dadurch gekennzeichnet, dass** eine Beschriftung vorhanden ist, die für die Freischaltung durch Code, das Update oder die zusätzliche Hardwarekomponente Austausch- oder Erweiterung ermöglicht, die ohne die Freischaltung durch Code, das Update oder die zusätzliche Hardwarekomponente nicht benötigt werden.

28. Beatmungsgerät nach einem der vorhergehenden Ansprüche, **dadurch gekennzeichnet, dass** definierte mehrmalige Eingabe eines oder mehrerer falscher Codes oder eines oder mehrerer nicht passender Updates zu einer zeitlich begrenzten oder unbegrenzten Sperre der Annahme jeder weiteren Eingabe für Codes oder Updates führt.

29. Beatmungsgerät nach Anspruch 28, **dadurch gekennzeichnet, dass** mit einem weiteren speziellen Code oder Update die Annahme wieder ermöglicht wird.

## Claims

1. Method for updating a ventilation device, in which an operating program stored in the device is replaced at least in part by a new operating program and the operating program is stored at least in part in a modifiable memory of the device and is replaced at least in part by the new operating program when the update is carried out, wherein
a respiratory gas pump is arranged and a connection tube (5) is connected by way of a coupling (4) in the ventilation device in the region of a device housing (1) with an operating field (2) and a display as an indicator (3), wherein
the device housing (1) has an interface (8) for facilitating a data transfer and wherein the interface (8) is realized as a USB interface, by means of which the input and/or output of data is effectuated, and, moreover, interfaces for adaptable add-on devices and information management systems, for example for receiving storage media or for the connection to an ECG, EEG, printer, defibrillator, pulse oximeter or any other medical-technical device, are provided in the device, and wherein
recorded data, such as trends, extraordinary events, warning notifications, are transmitted to the medical practitioner and conspicuous events, operating hours or other data for ensuring the flawless operation are transmitted to the user or the maintenance/customer service when required, in each case by means of a modem, and wherein
a pulse oximeter is adaptable to an interface for ascertaining further body parameters and the body parameters ascertained by the adapted pulse oximeter are displayed on the display of the ventilation device,
**characterized in that**
such regions which are only activated once the relevant sensor is adapted are provided in the region of the display and measurement values of the adapted pulse oximeter are then displayed in these regions.

2. Method according to Claim 1, **characterized in that** the new operating program is stored on a data medium and transferred to the memory by a read device.

3. Method according to Claim 1, **characterized in that** the new operating program is transferred online to the device.

4. Method according to any one of Claims 1 to 3,
**characterized in that** a base operating program which is not modifiable during the update is stored in the region of the device.

5. Method according to any one of Claims 1 to 4,
**characterized in that** an input code is compared to a reference value by a testing means of the device and at least one function of the device is only enabled if the code corresponds to the reference value.

6. Method according to any one of Claims 1 to 5,
**characterized in that** the new operating program activates hardware components which are already present in the device.

7. Method according to any one of Claims 1 to 6,
**characterized in that** the new operating program modifies a functionality of hardware components of the device which are already present.

8. Method according to any one of Claims 1 to 7,
**characterized in that** a positive comparison result is indicated or reported to the user in an acoustic, optical or any other manner.

9. Method according to any one of the preceding claims, **characterized in that** a negative comparison result is indicated or reported to the user in an acoustic, optical or any other manner.

10. Method according to any one of the preceding claims, **characterized in that** data are stored in the storage apparatus and/or in the code, said data (also in processed or calculated form) temporally restricting the execution of the functions and/or the blocking thereof in a defined manner.

11. Method according to any one of the preceding claims, **characterized in that** hardware components are contained in the device, the functions of said hardware components not being available without the code input and said hardware components being blocked for all functions in this respect.

12. Method according to any one of the preceding claims, **characterized in that**, without the code input, the supply of the device or of parts thereof with electrical energy (e.g. by way of a mains connection, batteries or accumulators) is prevented, or restricted in terms of time or power in a defined manner.

13. Method according to any one of the preceding claims, **characterized in that** the input or transmission of the code to the input apparatus is effectuated (also wirelessly) by means of a data medium with patient-related or practice-related or physician-related data (e.g. a health card) or via a read device to this end.

14. Method according to any one of the preceding claims, **characterized in that** the input or transmission of the code to the input apparatus is effectuated by means of an apparatus (also a wireless apparatus), which is internally or externally assigned, at least intermittently, to another medical, physician-related or health-related device or which, as an interface, connects a plurality of medical, physician-related or health-related devices by way of at least intermittent data or program interchange.

15. Method according to any one of the preceding claims, **characterized in that**, without the code input, certain components of the device, in particular sensors of specific characteristic data, are blocked at least intermittently from use for the device.

16. Ventilation device with an apparatus,
wherein the apparatus consists of an internal or external input apparatus for data input or data reception, of a storage apparatus which is at least indirectly connected thereto, with data or computational algorithms stored in the storage apparatus in a memory, and with testing means configured to automatically compare input or received data with data stored in a memory of the storage apparatus, wherein control means are present which, on account of the comparison result ascertained by the testing means, only in the case of certain defined updates independently cause the ventilation device to permanently or intermittently apply these data to hardware functions which are assigned to these updates, and cause the execution of these hardware functions assigned to the updates to be blocked by these control means or by other means without the appropriate update, wherein
a respiratory gas pump is arranged and a connection tube (5) is connected by way of a coupling (4) in a device interior in the ventilation device in the region of a device housing (1) with an operating field (2) and a display as an indicator (3), wherein
the device housing (1) has an interface (8) for facilitating a data transfer and wherein the interface (8) is realized as a USB interface which is configured to be able to effectuate the input and/or output of data, and wherein, moreover, interfaces for adaptable add-on devices and information management systems, for example for receiving storage media or for the connection to an ECG, EEG, printer, defibrillator, pulse oximeter or any other medical-technical device, are provided in the ventilation device, and
having a modem which is configured to be able to transmit recorded data, such as trends, extraordinary events, warning notifications, to the medical practitioner and conspicuous events, operating hours or other data for ensuring the flawless operation to the user or the maintenance/customer service when required, and wherein
a pulse oximeter is adaptable to an interface for ascertaining further body parameters and wherein the display is configured to display the body parameters ascertained by the adapted pulse oximeter,
**characterized in that**
such regions which are only activated once the relevant sensor is adapted are provided in the region of the display and measurement values of the adapted pulse oximeter are then displayed in these regions.

17. Ventilation device according to any one of the preceding claims, **characterized in that** hardware components are contained in the ventilation device, the functions of said hardware components not being available without the update and said hardware components being blocked for all functions in this respect.

18. Ventilation device according to any one of the preceding claims, **characterized in that**, without the update, the supply of the ventilation device or of parts thereof with electrical energy (e.g. by way of a mains connection, batteries or accumulators) is prevented, or restricted in terms of time or power in a defined manner.

19. Ventilation device according to any one of the preceding claims, **characterized in that** the input or transmission of the update to the input apparatus is effectuated (also wirelessly) by means of a data medium with patient-related or practice-related or physician-related data (e.g. a health card) or via a read device to this end.

20. Ventilation device according to any one of the preceding claims, **characterized in that** the input or transmission of the update to the input apparatus is effectuated by means of an apparatus, for example a wireless apparatus, which is internally or externally assigned, at least intermittently, to another medical, physician-related or health-related device or which, as an interface, connects a plurality of medical, physician-related or health-related devices by way of at least intermittent data or program interchange.

21. Ventilation device according to any one of the preceding claims, **characterized in that**, without the update, certain components of the device, in particular sensors of specific characteristic data, are blocked at least intermittently from use for the device.

22. Ventilation device according to Claim 21, **characterized in that** the characteristic data are the wavelengths of the sensors.

23. Ventilation device according to any one of the preceding claims, **characterized in that** hardware or software components are already contained in the ventilation device, the functions of said hardware or software components not being available without the cooperation of the device with the assigned additional hardware component and said hardware or software components being blocked for all functions in this respect.

24. Ventilation device according to any one of the preceding claims, **characterized in that**, without the cooperation of the device with the assigned additional hardware component, the supply of the device or of parts thereof with electrical energy (e.g. by way of a mains connection, batteries or accumulators) is prevented, or restricted in terms of time or power in a defined manner.

25. Ventilation device according to Claim 24, **characterized in that** the already present hardware components which are blocked without the code are defined sensors, in particular of specific wavelengths.

26. Ventilation device according to any one of the preceding claims, **characterized in that** an optical indicator is present, which, for the enablement by code, the update or the additional hardware component, keeps available indication options which cannot be used, without the enablement by code, the update or the additional hardware component.

27. Ventilation device according to any one of the preceding claims, **characterized in that** an annotation is present which, for the enablement by code, the update or the additional hardware component, facilitates interchange or expansion, which are not required without the enablement by code, the update or the additional hardware component.

28. Ventilation device according to any one of the preceding claims, **characterized in that** defined multiple input of one or more incorrect codes or one or more inappropriate updates leads to a time-restricted or non-restricted block from receiving any further input for codes or updates.

29. Ventilation device according to Claim 28, **characterized in that** a further special code or update facilitates the reception again.

## Revendications

1. Procédé d'actualisation d'un appareil respiratoire, avec lequel un programme d'exploitation enregistré dans l'appareil est au moins partiellement remplacé par un nouveau programme d'exploitation et le programme d'exploitation est au moins partiellement enregistré dans une mémoire modifiable de l'appareil et est au moins partiellement remplacé par le nouveau programme d'exploitation lors de l'exécution de l'actualisation,
dans l'appareil respiratoire, dans la zone d'un boîtier d'appareil (1) comprenant un panneau de commande (2) ainsi qu'un afficheur faisant office d'indicateur (3), une pompe à gaz respiratoire étant disposée dans un espace intérieur d'appareil et un tuyau de liaison (5) étant raccordé par le biais d'un coupleur (4),
le boîtier d'appareil (1) possédant une interface (8) destinée à rendre possible une transmission de données et l'interface (8) étant réalisée sous la forme d'une interface USB par le biais de laquelle s'effectue la saisie et/ou la diffusion de données et, en plus de cela, des interfaces pour des appareils supplémentaires adaptables et des systèmes de gestion d'informations, par exemple destinés à accueillir des supports d'enregistrement ou destinés à la connexion avec un ECG, un EEG, une imprimante, un défibrillateur, un oxymètre de pouls ou un autre appareil technique médical étant présents dans l'appareil et,
par le biais d'un modem, en cas de besoin, des données enregistrées, comme des tendances, des événements inhabituels, des avertissements étant communiqués au médecin ; ainsi que des anomalies, des heures de fonctionnement ou d'autres données destinées à garantir le bon fonctionnement à l'utilisateur ou au service de maintenance/clientèle et
un oxymètre de pouls étant adaptable sur une interface pour la détermination de paramètres corporels supplémentaires et les paramètres corporels déterminés par l'oxymètre de pouls étant affichés sur l'afficheur de l'appareil respiratoire,
**caractérisé en ce que** dans la zone de l'afficheur se trouvent des zones qui ne sont activées que lorsque le capteur concerné est adapté et les valeurs mesurées de l'oxymètre de pouls adapté sont alors affichées dans ces zones.

2. Procédé selon la revendication 1, **caractérisé en ce que** le nouveau programme d'exploitation est enregistré sur un support de données et transmis à la mémoire par un lecteur.

3. Procédé selon la revendication 1, **caractérisé en ce que** le nouveau programme d'exploitation est transmis à l'appareil en ligne.

4. Procédé selon l'une des revendications 1 à 3, **caractérisé en ce qu'**un programme d'exploitation de base qui n'est pas modifiable lors de l'actualisation est enregistré dans la zone de l'appareil.

5. Procédé selon l'une des revendications 1 à 4, **caractérisé en ce qu'**un code saisi est comparé à une valeur de référence par un moyen de contrôle de l'appareil et au moins une fonction de l'appareil n'est libérée que dans le cas d'une concordance du code avec la valeur de référence.

6. Procédé selon l'une des revendications 1 à 5, **caractérisé en ce que** des composants matériels déjà présents dans l'appareil sont activés par le nouveau programme d'exploitation.

7. Procédé selon l'une des revendications 1 à 6, **caractérisé en ce qu'**une fonctionnalité de composants matériels de l'appareil déjà présents est modifiée par le nouveau programme d'exploitation.

8. Procédé selon l'une des revendications 1 à 7, **caractérisé en ce qu'**un résultat de comparaison positif est affiché ou signalé à l'utilisateur de manière sonore, visuelle ou sous une autre forme.

9. Procédé selon l'une des revendications précédentes, **caractérisé en ce qu'**un résultat de comparaison négatif est affiché ou signalé à l'utilisateur de manière sonore, visuelle ou sous une autre forme.

10. Procédé selon l'une des revendications précédentes, **caractérisé en ce que** dans le dispositif de mémorisation et/ou dans le code sont stockées des données qui (également sous une forme traitée ou calculée) limitent l'exécution des fonctions et/ou leur blocage dans le temps d'une manière définie.

11. Procédé selon l'une des revendications précédentes, **caractérisé en ce que** l'appareil contient des composants matériels qui, en l'absence de la saisie du code, ne peuvent pas être mis en fonction et, dans ce contexte, sont bloqués pour toutes les fonctions.

12. Procédé selon l'une des revendications précédentes, **caractérisé en ce qu'**en l'absence de la saisie du code, l'alimentation de l'appareil ou de parties de celui-ci en énergie électrique (par exemple par le biais d'une connexion au réseau, de piles ou de batteries) est inhibée ou limitée d'une manière définie dans le temps ou au niveau de la puissance.

13. Procédé selon l'une des revendications précédentes, **caractérisé en ce que** la saisie ou la communication du code au dispositif d'entrée s'effectue au moyen d'un support de données comprenant des données relatives au patient, au cabinet ou au médecin (par exemple une carte de santé) ou par le biais d'un lecteur conçu à cet effet (également sans fil).

14. Procédé selon l'une des revendications précédentes, **caractérisé en ce que** la saisie ou la communication du code au dispositif d'entrée s'effectue par un dispositif (également sans fil) qui est associé au moins temporairement en interne ou en externe à un autre appareil médical, médicinal ou en rapport avec la santé ou qui, en tant qu'interface, relie plusieurs appareils médicaux, médicinaux ou en rapport avec la santé par le biais d'un échange au moins temporaire de données ou de programme.

15. Procédé selon l'une des revendications précédentes, **caractérisé en ce qu'**en l'absence de la saisie du code, certains éléments structuraux de l'appareil, notamment des capteurs de certaines données caractéristiques, sont au moins temporairement bloqués en vue d'une utilisation pour l'appareil.

16. Appareil respiratoire comprenant un dispositif, le dispositif se composant d'un dispositif d'entrée interne ou externe pour la saisie de données ou la réception de données, depuis un dispositif de mémorisation connecté au moins indirectement à celui-ci, comprenant des données ou des algorithmes de calcul stockés dans une mémoire dans le dispositif de mémorisation, et comprenant également des moyens de contrôle qui sont conçus pour comparer automatiquement des données saisies ou reçues avec des données stockées dans une mémoire du dispositif de mémorisation, des moyens de commande étant présents qui, sur la base du résultat de la comparaison déterminé par les moyens de contrôle, n'amènent automatiquement que dans le cas de certaines mises à jour définies l'appareil respiratoire à utiliser en permanence ou temporairement ces données sur des fonctions matérielles qui sont associées à ces mises à jour, et que l'exécution de ces fonctions matérielles associées aux mises à jour est bloquée par ces moyens de commande ou par d'autres moyens en l'absence de la mise à jour correspondante,
dans l'appareil respiratoire, dans la zone d'un boîtier d'appareil (1) comprenant un panneau de commande (2) ainsi qu'un afficheur faisant office d'indicateur (3), une pompe à gaz respiratoire étant disposée dans un espace intérieur d'appareil et un tuyau de liaison (5) étant raccordé par le biais d'un coupleur (4),
le boîtier d'appareil (1) possédant une interface (8) servant à rendre possible une transmission de données et l'interface (8) étant réalisée sous la forme d'une interface USB, laquelle est conçue pour faire effectuer la saisie et/ou la diffusion de données et, en plus de cela, des interfaces pour des appareils supplémentaires adaptables et des systèmes de gestion d'informations, par exemple destinées à accueillir des supports d'enregistrement ou destinées à la connexion avec un ECG, un EEG, une imprimante, un défibrillateur, un oxymètre de pouls ou un autre appareil technique médical, étant présentes dans l'appareil respiratoire et,
comprenant un modem qui est conçu pour pouvoir communiquer en cas de besoin des données enregistrées, comme des tendances, des événements inhabituels, des avertissements au médecin ; ainsi que des anomalies, des heures de fonctionnement ou d'autres données destinées à garantir le bon fonctionnement à l'utilisateur ou au service de maintenance/clientèle et un oxymètre de pouls étant adaptable sur une interface pour la détermination de paramètres corporels supplémentaires et l'afficheur étant conçu pour afficher les paramètres corporels déterminés par l'oxymètre de pouls adapté,
**caractérisé en ce que** dans la zone de l'afficheur se trouvent des zones qui ne sont activées que lorsque le capteur concerné est adapté et des valeurs mesurées de l'oxymètre de pouls adapté sont alors affichées dans ces zones.

17. Appareil respiratoire selon l'une des revendications précédentes, **caractérisé en ce que** l'appareil respiratoire contient des composants matériels qui, en l'absence de la mise à jour, ne peuvent pas être mis en fonction et, dans ce contexte, sont bloqués pour toutes les fonctions.

18. Appareil respiratoire selon l'une des revendications précédentes, **caractérisé en ce qu'**en l'absence de la mise à jour, l'alimentation de l'appareil respiratoire ou de parties de celui-ci en énergie électrique (par exemple par le biais d'une connexion au réseau, de piles ou de batteries) est inhibée ou limitée d'une manière définie dans le temps ou au niveau de la puissance.

19. Appareil respiratoire selon l'une des revendications précédentes, **caractérisé en ce que** la saisie ou la communication de la mise à jour au dispositif d'entrée s'effectue au moyen d'un support de . données comprenant des données relatives au patient, au cabinet ou au médecin (par exemple une carte de santé) ou par le biais d'un lecteur conçu à cet effet (également sans fil).

20. Appareil respiratoire selon l'une des revendications précédentes, **caractérisé en ce que** la saisie ou la communication de la mise à jour au dispositif d'entrée s'effectue par un dispositif, par exemple sans fil, qui est associé au moins temporairement en interne ou en externe à un autre appareil médical, médicinal ou en rapport avec la santé ou qui, en tant qu'interface, relie plusieurs appareils médicaux, médicinaux ou en rapport avec la santé par le biais d'un échange au moins temporaire de données ou de programme.

21. Appareil respiratoire selon l'une des revendications précédentes, **caractérisé en ce qu'**en l'absence de la mise à jour, certains éléments structuraux de l'appareil, notamment des capteurs de certaines données caractéristiques, sont au moins temporairement bloqués en vue d'une utilisation pour l'appareil.

22. Appareil respiratoire selon la revendication 21, **caractérisé en ce que** les données caractéristiques sont les longueurs d'onde des capteurs.

23. Appareil respiratoire selon l'une des revendications précédentes, **caractérisé en ce que** l'appareil respiratoire contient déjà des composants matériels ou logiciels qui, en l'absence de la coopération de l'appareil avec les composants matériels supplémentaires correspondants, ne peuvent pas être mis en fonction et, dans ce contexte, sont bloqués pour toutes les fonctions.

24. Appareil respiratoire selon l'une des revendications précédentes, **caractérisé en ce qu'**en l'absence de la coopération de l'appareil avec les composants matériels supplémentaires correspondants, l'alimentation de l'appareil ou de parties de celui-ci en énergie électrique (par exemple par le biais d'une connexion au réseau, de piles ou de batteries) est inhibée ou limitée d'une manière définie dans le temps ou au niveau de la puissance.

25. Appareil respiratoire selon la revendication 24, **caractérisé en ce que** les composants matériels déjà présents qui sont bloqués en l'absence de code sont des capteurs définis, notamment de longueurs d'onde spécifiques.

26. Appareil respiratoire selon l'une des revendications précédentes, **caractérisé en ce qu'**un indicateur visuel est présent, lequel met à disposition des possibilités d'affichage pour la libération par le code, la mise à jour ou les composants matériels supplémentaires, lesquelles ne peuvent pas être utilisées sans la libération par le code, la mise à jour ou les composants matériels supplémentaires.

27. Appareil respiratoire selon l'une des revendications précédentes, **caractérisé en ce qu'**un marquage est présent, lequel rend possible un échange ou une extension pour la libération par le code, la mise à jour ou les composants matériels supplémentaires, lesquels ne sont pas nécessaires en l'absence de la libération par le code, la mise à jour ou les composants matériels supplémentaires.

28. Appareil respiratoire selon l'une des revendications précédentes, **caractérisé en ce que** saisie multiple définie d'un ou plusieurs codes erronés ou d'une ou plusieurs mises à jour non adaptées se traduit par un blocage limité ou illimité dans le temps de l'acceptation de toute saisie supplémentaire pour des codes ou des mises à jour.

29. Appareil respiratoire selon la revendication 28, **caractérisé en ce que** l'acceptation est de nouveau rendue possible avec un code ou une mise à jour supplémentaire spécial.
